# EUROPÄISCHE PATENTSCHRIFT

(11) **EP 4 140 472 B1**
(45) Veröffentlichungstag und Bekanntmachung des Hinweises auf die Patenterteilung: **22.10.2025**
(21) Anmeldenummer: 22190992.2
(22) Anmeldetag: 27.01.2020
(51) Int. Cl.: A61K 8/60, A61Q 19/10, A61K 8/42, A61K 8/44

(54) **GLYCOLIPID-HALTIGE REINIGUNGSZUBEREITUNG ENTHALTEND MIZELLEN**
GLYCOLIPID-CONTAINING CLEANSING PREPARATION CONTAINING MICELLES
COMPOSITION NETTOYANTE CONTENANT DES GLYCOLIPIDES, CONTENANT DES MICELLES

(30) Priorität: 28.02.2019 DE 102019202724
(43) Veröffentlichungstag der Anmeldung: 01.03.2023
(62) Teilanmeldung aus: 20702759.0
(73) Patentinhaber: Beiersdorf AG, 22529 Hamburg (DE)
(72) Erfinder: Vetter, Katrin, 23843 Bad Oldesloe (DE); Raschke, Thomas, 25421 Pinneberg (DE); Demitz, Kay Sven, 22529 Hamburg (DE); Spille, Sandra, 22769 Hamburg (DE)
(74) Vertreter: Beiersdorf AG

(56) Entgegenhaltungen:
- EP-A1- 1 445 302
- WO-A1-2011/051161
- WO-A1-2011/120776
- WO-A2-2004/108063
- DE-A1- 102015 217 507
- US-A1- 2013 287 708

## Beschreibung

Die vorliegende Erfindung betrifft eine Reinigungszubereitung, die Glycolipide in einer Menge enthält, so dass sich Mizellen ausbilden können.

Diese Zubereitung ist bevorzugt dazu geeignet, Make-up von der menschlichen Haut, insbesondere der Gesichtshaut zu entfernen.

Die Verwendung von Make-up zur Verschönerung der äußeren Erscheinung, insbesondere des Gesichts, ist ein Phänomen, das schon lange zu beobachten ist. Bereits im alten Ägypten wurden Kajal, Lidschatten, Rouge und Lippenstift verwendet, und zwar von Männern und Frauen. Neben einer Schutzfunktion hatte das Schminken auch kosmetische Effekte. Make-up wurde einfach um der Schönheit willen getragen. Dies gilt auch heute noch.

Das verwendete Make-up muss auch wieder entfernt werden. Zur Entfernung von Make-up aus dem Gesichtsbereich gibt es eine besondere Gruppe von Hautreinigungsprodukten, nämlich Gesichtsreinigungsprodukte. Da die Gesichtshaut besonders empfindlich ist, werden für die Gesichtsreinigung besonders milde und die Haut nicht reizende Produkte eingesetzt.

Im Bereich der dekorativen Kosmetik finden heutzutage eine Vielzahl von unterschiedlichen Stoffen Anwendung. Als Farbstoffe werden neben anorganischen Pigmenten wie Silikaten [Magnesiumsilikat (Talkum), Aluminiumsilikat (Kaolin)] und Metalloxiden (Chrom-, Eisen-, Mangan-, Titan- und Zinkoxiden) organische Farbpigmente eingesetzt. Als Bindemittel finden unter anderem Stearinsäureester, Lanolinalkohol und -acetat Verwendung. In vielen Formulierungen werden Wachse wie Bienenwachs oder Carnaubawachs und Öle wie Paraffinöle, Silikonöle oder Ricinusöl eingesetzt. Des Weiteren können dekorative Kosmetika Konservierungsstoffe, Antioxidantien, Verdickungsmittel und andere Zusätze enthalten.

Um diese Vielzahl völlig unterschiedlicher Stoffe von der Haut zu entfernen, bedarf es entsprechender kosmetischer Reinigungsmittel. Sie müssen unpolare Verbindungen wie Wachse, Öle und Silikonverbindungen lösen und gleichzeitig die schwerlöslichen Pigmente wie Talkum oder Titandioxid aufnehmen. Dies gilt insbesondere für Wimperntusche, Mascara, Lidschatten und Kajalstifte. Andererseits müssen sie so hautverträglich wie möglich sein, um bei den Anwendern keine Hautrötungen oder Schleimhautirritationen auszulösen.

Im Stand der Technik wurden bereits Zubereitungen offenbart, die zur Make-up-Entfernung geeignet sind oder speziell für diesen Zweck entwickelt wurden. Verschiedenartige Zubereitungsformen wurden beschrieben und entsprechende Produkte sind bereits käuflich zu erwerben. Diese Zubereitungen können in Form von Emulsionen, die wiederum überwiegend cremig, milchig oder flüssig sind, Gelen oder öligen Zusammensetzungen vorliegen. Weiterhin sind mehrphasige Zusammensetzungen bekannt, insbesondere zweiphasige Zusammensetzungen, die zwei visuell getrennte Phasen aufweisen, die durch Schütteln gemischt werden. Die entstandene weitgehend homogene Phase wird dann auf die Haut aufgetragen.

WO 2016/146360 A1 offenbart eine Gesichts-Reinigungszubereitung, die geeignet ist, Make-up zu entfernen und gleichzeitig die Haut zu erfrischen. Diese Zubereitung weist eine wässrige und eine ölige Phase auf und ist frei von Silikonverbindungen.

WO 2017/005485 A1 beschreibt ein Tränkungsmittel für Tücher. Das entsprechende Reinigungstuch ist für die Gesichtsreinigung vorgesehen. Das Tränkungsmittel basiert auf der Mizellentechnologie und enthält eine Kombination von Tensiden, nämlich anionische Tenside in Form von Acylglutamaten und nichtionische Tenside in Form von Alkylglycosiden und Poloxameren.

Weiterhin sind dem Fachmann die Schriften WO2004/108063 A2, US2013/287708 A1, WO2011/120776 A1, DE102015217507 A1, EP 1445302 A1 und WO2011/051161 A1 bekannt, doch konnten diese Veröffentlichungen nicht den Weg zur vorliegenden Erfindung weisen.

Es besteht aber weiterhin Bedarf, die Zubereitungen, die für die Gesichtsreinigung vorgesehen sind und insbesondere Make-up entfernen sollen, zu verbessern. Verbesserungen können und sollen in zweierlei Hinsicht erzielt werden. Zum einen soll die Reinigungsleistung verbessert werden, d.h. insbesondere die Make-up-Entfernung soll schnell und sicher erfolgen. Zum anderen soll auch die Milde der Zubereitungen weiter verbessert werden. Wie aus dem Stand der Technik ersichtlich, enthalten Reinigungszubereitungen für die Gesichtsreinigung und insbesondere Make-up-Entfernung Tenside, meist Kombinationen von Tensiden. Es gibt verschiedene Möglichkeiten der Gesichtsreinigung. Sogenannte Waschgele, die Tenside in Konzentrationen von etwa 3 bis 10 % enthalten, müssen abgespült werden. Eine weitere Möglichkeit sind Zubereitungen, die deutlich weniger Tenside enthalten und somit nicht abgespült werden müssen. Auch sogenannte

Mizellenwasser gehören zu dieser Art Zubereitungen. Sie zeichnen sich dadurch aus, dass das/die Tensid(e) in einer derartigen Konzentration vorliegt/en, dass sich Tensidmizellen ausbilden können. Die Mizellen sind meist so angeordnet, dass die lipophilen Teile der Tenside ins Innere der Mizelle gerichtet sind und die hydrophilen Teile der Tenside nach außen ins wässrige Milieu zeigen. Die Mizellen können mit den Molekülen auf der Hautoberfläche wechselwirken. Diese Moleküle können aus Make-up oder Make-up-Resten, Talg oder auch abgestorbene Hautzellen und anderem stammen. Auf diese Weise tragen die Mizellen zur Solubilisierung dieser Moleküle bei. Mithilfe eines Wattepads oder ähnlichem oder mit Hilfe von Wasser können nun diese Moleküle, d.h. der Schmutz von der Haut entfernt werden. In der Regel weisen die Mizellenwasser des Standes der Technik einen geringeren Tensidgehalt auf als klassische Reinigungszubereitungen auf. Trotz des vergleichsweise geringen Tensidgehaltes können bei derartigen Zubereitungen Unverträglichkeiten beobachtet werden, wie beispielsweise Reizungen der Haut (z.B. Rötungen, Spannungsgefühle) oder ein Brennen der Augen.

Aufgabe der vorliegenden Erfindung war es, Verbesserungen herbeizuführen, d. h. Unverträglichkeitsreaktionen zu minimieren, idealerweise zu unterbinden und gleichzeitig eine gute bis sehr gute Reinigungsleistung zu erbringen.

Überraschend wurde gefunden, dass eine Reinigungszubereitung gemäß Anspruch 1 die Aufgabe der vorliegenden Erfindung zu lösen vermag.

Die erfindungsgemäße Zubereitung ist eine wässrige Zubereitung.

Die erfindungsgemäße Zubereitung ist eine kosmetische Zubereitung. Die erfindungsgemäße Zubereitung wird vorteilhaft zur Reinigung der menschlichen Haut, insbesondere der Gesichtshaut eingesetzt.

In der erfindungsgemäßen Zubereitung sind vorteilhaft Mizellen, insbesondere Tensidmizellen enthalten.

Die erfindungsgemäße Zubereitung enthält Biotenside.

Tenside sind amphiphile Stoffe, die u.a. organische, unpolare Substanzen in Wasser zu lösen vermögen. Sie zeichnen sich durch ein ambivalentes Verhalten gegenüber Wasser und Lipiden aus. Das Tensidmolekül enthält mindestens je eine hydrophile und eine lipophile Gruppe, die die Anlagerung an der Grenzfläche zwischen einer wässrigen Phase und einer Lipidphase ermöglichen. Auf diese Weise sorgen Tenside für eine Herabsetzung der Oberflächenspannung des Wassers, die Benetzung der Haut, die Erleichterung der Schmutzlösung und -entfernung, ein leichtes Abspülen und optional auch für Schaumregulierung. Damit ist die Grundlage für die Schmutzentfernung lipidhaltiger Verschmutzungen gegeben.

Unter Biotensiden werden Tenside verstanden, die aus nachwachsenden Rohstoffen gewonnen werden und biologisch abbaubar sind. Der Herstellungsprozess dieser Tenside ist ebenfalls nachhaltig, idealerweise mithilfe eines Fermentationsprozesses. Als Biotenside sind Glycolipide, Lipopeptide, Fettsäuren, Phospholipide und andere zu nennen. Erfindungsgemäß sind Glycolipide, eine Gruppe die Rhamnolipide, Sophorolipide, Mannosylerythrollipide und Trehaloselipide umfasst. Bevorzugt sind Rhamnolipide.

Rhamnolipide lassen sich durch die folgende Strukturformel beschreiben: 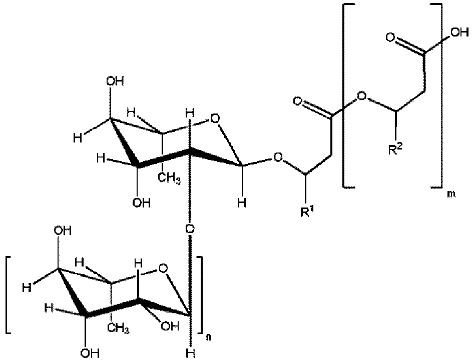
wobei m = 1 oder 0 ist,
n = 1 oder 0 ist,
R¹ und R² unabhängig voneinander ein gleicher oder verschiedener organischer Rest mit 2 bis 30, bevorzugt 5 bis 24 Kohlenstoffatomen ist, insbesondere ein substituierter oder unsubstituierter, verzweigter oder unverzweigter Alkylrest, der auch ungesättigt sein kann,
wobei der Alkylrest bevorzugt ein linearer gesättigter Alkylrest mit 6 bis 20 Kohlenstoffatomen ist. Salze dieser Verbindungen sind erfindungsgemäß ebenfalls umfasst. Unter dem Begriff "di-Rhamnolipid" werden Verbindungen der obenstehenden Formel oder deren Salze verstanden, bei denen n = 1 ist, also zwei Rhamnose-Einheiten enthalten sind. Entsprechend werden unter "mono-Rhamnolipid" Verbindungen der obenstehenden Formel oder deren Salze verstanden, bei denen n = 0 ist, es ist also nur eine Rhamnose-Einheit enthalten.

Rhamnolipide können beispielsweise durch Biosynthese in Bakterien der Gattung Pseudomonas, insbesondere Pseudomonas aeruginosa erhalten werden.

Der Einsatz von Biotensiden, insbesondere auch Rhamnolipiden, ist bereits beschrieben worden.

DE 19600743 A1 beschreibt den Einsatz von Glycolipiden und weiteren Tensiden in Geschirrspülmitteln.

DE 102015217503 A1 offenbart den Einsatz von Biotensiden in kosmetischen Reinigungsmitteln, wobei als weitere Tenside anionische Tenside enthalten sind.

WO 2014/173659 A1 beschreibt die Verwendung von Glycolipiden in Reinigungszubereitungen. Auch in diesen Reinigungszubereitungen muss ein weiteres Tensid enthalten sein, nämlich ein synthetisches Tensid.

DE 102015217504 A1 offenbart Peeling-Zusammensetzungen, die neben dem Peelingmittel Biotenside und einen Verdicker enthalten müssen.

Das Dokument WO 2013/098066 A2 beschreibt Haar- und Hautreinigungszusammensetzungen, die Biotenside enthalten. Weiterhin ist mindestens immer eine Fettsäure enthalten.

Im Dokument EP 2786742 A1 wird die Verwendung von Rhamnolipiden in Kosmetika beschrieben. Alle Ausführungsbeispiele enthalten wenigstens noch ein weiteres Tensid oder einen Emulgator.

Erfindungsgemäß vorteilhaft kann das wenigstens eine Rhamnolipid unter Handelsbezeichnung Rheance One von der Firma Evonik bezogen werden.

In der erfindungsgemäßen Zubereitung liegt das wenigstens eine Glycolipid, insbesondere das wenigstens eine Rhamnolipid, mit einem Gehalt von 0,007 bis 0,8 Gew.-%, bevorzugt von 0,01 bis 0,5 Gew.%, besonders bevorzugt 0,05 bis 0,3 Gew.-% vor, bezogen auf die Gesamtmenge des Zubereitung und bezogen auf den Aktivgehalt.

Aus der Gruppe der anionischen Tenside werden Tenside ausgewählt, die auf Aminosäuren basieren, also Aminosäure-basierte Tenside.

Geeignete Aminosäure-basierte Tenside (und/oder deren Salze) können beispielsweise ausgewählt werden aus:
1. Acylglutamate, beispielsweise Natriumacylglutamat wie beispielsweise Natrium Cocoylglutamat und
2. Glycinate, wie beispielsweise Acyl-Glycinate.

Es ist bevorzugt, wenn ein oder mehrere Acylglutamat(e) und/oder Acylglycinat(e) enthalten sind.

Ein besonders vorteilhaftes Acylglutamat ist Disodium Cocoyl Glutamate, das beispielsweise mit dem Handelsnamen Hostapon - CCG von der Firma Clariant erhältlich ist. Ebenso besonders vorteilhaft ist das Acylglycinat mit der Bezeichnung Natrium Cocoyl Glycinat, das beispielsweise unter dem Handelsnamen Hostapon SG bei der Firma Clariant erhältlich ist.

Wenn die erfindungsgemäße Zubereitung ein oder mehrere Tenside enthält, die auf Aminosäuren basieren, so liegt der Gesamtgehalt des genannten einen Tensids oder der genannten mehreren Tenside im Bereich von 0,001 bis 0,5 Gew.-%, bevorzugt 0,05 bis 0,3 Gew.-%, bezogen auf das Gesamtgewicht der Zubereitung und den jeweiligen Aktivgehalt.

Aus der Gruppe der amphoteren Tenside können auch vorteilhafte Vertreter ausgewählt werden. Amphotere Tenside weisen sowohl anionische als auch kationische Gruppen auf und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:
RNH₂⁺-CH₂-CH₂-COOH X⁻ (bei pH=2) X⁻ = beliebiges Anion, z.B. Cl⁻
RNH₂⁺-CH₂-CH₂-COO⁻ (bei pH=7)
RNH-CH₂-CH₂-COO⁻ B⁺ (bei pH=12) B⁺ = beliebiges Kation, z.B. Na⁺.

Geeignete amphotere Tenside können beispielsweise ausgewählt werden aus:
1. Acyl-/dialkylethylendiamin, beispielsweise Natriumacylamphoacetat, Dinatriumacylamphodipropionat, Dinatriumalkylamphodiacetat, Dinatrium Cocoamphodiacetat, Dinatrium Cocoamphomonoacetat, Natriumacylamphohydroxypropylsulfonat, Dinatriumacylamphodiacetat und Natriumacylamphopropionat,
2. Betaine, beispielsweise Coco Betaine, Cocoamidopropyl Betaine,
3. Sultaine, beispielsweise Lauryl Hydroxy Sultaine.

Es ist bevorzugt, wenn das oder die amphotere(n) Tensid(e) ausgewählt werden aus der Gruppe der Betaine, weiter bevorzugt aus der Gruppe Alkylamidopropylbetaine und Alkylbetaine. Es ist besonders bevorzugt, wenn das amphotere Tensid Cocamidopropyl Betain ist.

Es ist ebenso bevorzugt, wenn das oder die amphotere(n) Tensid(e) ausgewählt werden aus der Gruppe der Sultaine, weiter bevorzugt aus der Gruppe Hydroxysultaine und/oder Amidopropylhydroxysultaine.

Weiterhin ist ebenso bevorzugt, wenn das oder die amphoteren Tenside ausgewählt werden aus Acyl-/dialkylethylendiamin, insbesondere aus Acylamphoacetaten, die durch folgende Formel beschrieben werden können: 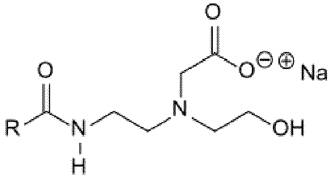 wobei R ein Alkylrest mit 8 bis 30 Kohlenstoffatomen ist.

Im Allgemeinen werden Acylamphoacetate in zwei Schritten synthetisiert. Zunächst wird eine Fettsäure oder ein Gemisch von Fettsäuren, die aus einem natürlichen Öl stammen, bevorzugt ein Pflanzenöl, wie beispielsweise Kokosöl, Sojaöl oder Palmöl, mit N-(2-Hydroxyethyl)ethylenediamine versetzt. Die entstandenen 2-Alkyl-1-Hydroxyethylimidazoline werden mit NaOH zu linearen Amidoaminen hydrolysiert. Diese werden wiederum mit Monochloressigsäure versetzt. Es entstehen Acylmonoamphoacetate. Werden größere molare Mengen an Monochloressigsäure eingesetzt entstehen Acylamphodiacetate.

Acylamphacetate sind amphotere Tenside, die je nach pH-Wert als anionische, zwitterionische oder kationische Tenside vorliegen. Die Natriumsalze der Acylamphoacetate sind bekannt dafür, dass sie wenig irritativ wirken.

Erfindungsgemäß ist es bevorzugt, wenn der Acylrest aus Fettsäuren pflanzlichen Ursprungs stammt, bevorzugt aus Fettsäuren, die aus Kokosöl gewonnen werden.

Weiterhin ist es bevorzugt, wenn Natriumsalze der Acylamphoacetate ausgewählt werden. Es ist besonders bevorzugt, wenn Natrium Cocoamphacetate in der erfindungsgemäßen Zubereitung enthalten ist

Wenn die erfindungsgemäße Zubereitung ein oder mehrere amphotere Tenside enthält, so liegt der Gesamtgehalt des wenigstens einen amphoteren Tensids im Bereich von 0,001 bis 0,5 Gew.-%, bevorzugt 0,05 bis 0,3 Gew.-%, bezogen auf das Gesamtgewicht der Zubereitung und den jeweiligen Aktivgehalt.

Aus der Gruppe der nichtionischen Tenside können auch vorteilhafte Vertreter ausgewählt werden. Nichtionische Tenside zeichnen sich dadurch aus, dass sie im wässrigen Medium keine Ionen ausbilden.

Geeignete nichtionische Tenside können beispielsweise ausgewählt werden aus:
1. Ether, beispielsweise Alkylpolyglycoside, wie Laurylglucosid, Decylglucosid und Cocoglucosid,
2. Polyglycerinester.

Es ist bevorzugt, wenn die erfindungsgemäße Zubereitung wenigstens ein nichtionisches Tensid ausgewählt aus Alkylpolyglycosiden enthält. Alkylpolyglycoside können durch die allgemeine Strukturformel RO-(Z)ₓ dargestellt werden, wobei R ein Alkylrest mit 6 bis 30 C-Atomen, Z ein Zuckerrest und x die Anzahl der Zuckerreste ist. Der Alkylrest kann ein Rest mit einer spezifischen Anzahl an C-Atomen sein oder kann eine Mischung von Alkylresten sein. Derartige Mischungen werden erhalten, wenn natürliche Öle wie beispielsweise Palmöl, Sojaöl oder Kokosöl als Ausgangsmaterial für die Herstellung der Alkylpolyglucoside verwendet werden. Es ist bevorzugt, wenn die Alkylgruppen im Wesentlichen 12 bis 18 C-Atome aufweisen.

Die Zuckerreste können Monosaccharide oder Oligosaccharide sein, die bevorzugt Zuckerreste mit 5 oder 6 C-Atomen aufweisen. Geeignete Zuckerreste sind beispielsweise Glucose, Fructose, Galaktose, Arabinose, Ribose, Xylose, Allose, Mannose, Gulose, Idose, Talose und Saccharose. Besonders bevorzugt sind Glucose und Saccharose, wobei Glucose insbesondere bevorzugt ist.

Alkylpolyglycoside enthalten im Durchschnitt 1,1 bis 5, bevorzugt 1,1 bis 2,0 Zuckerreste pro Molekül.

Ganz insbesondere bevorzugt sind Alkylpolyglycoside mit der Bezeichnung Decyl Glucoside, beispielsweise erhältlich mit dem Handelsnamen Plantacare 2000 UP von der Firma BASF Personal Care & Nutrition oder mit der Bezeichnung Coco-Glucoside, beispielsweise erhältlich mit dem Handelsnamen Plantacare 818 UP von der Firma BASF Personal Care & Nutrition.

Wenn die erfindungsgemäße Zubereitung nichtionische Tenside enthält, so liegt der Gesamtgehalt des wenigstens einen nichtionischen Tensids im Bereich von 0,001 bis 0,5 Gew.-%, bevorzugt 0,05 bis 0,3 Gew.-%, bezogen auf das Gesamtgewicht der Zubereitung und den jeweiligen Aktivgehalt.

Um die Ausbildung von Mizellen zu gewährleisten, müssen die Tenside in bestimmten Konzentrationen in der erfindungsgemäßen Zubereitung vorliegen. Die Zubereitungen enthalten also Tenside in einer Gesamttensidmenge von ≤ 0,8 Gew. % ist, bezogen auf die Gesamtmenge der Zubereitung. Diese Angabe bezieht sich auf Tenside, die einzeln in der Zubereitung vorliegen, als auch auf Kombinationen von Tensiden. Wenn in der erfindungsgemäßen Zubereitung wenigstens eine Glycolipid, insbesondere Rhamnolipid und wenigstens ein weiteres Tensid vorliegen, so liegt auch in diesen Ausführungsformen die Gesamttensidmenge bei ≤ 0,8 Gew. %, bezogen auf die Gesamtmenge der Zubereitung.

Die erfindungsgemäße Zubereitung kann vorteilhaft Konservierungsmittel enthalten. Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Das wenigstens eine Konservierungsmittel kann vorteilhaft ausgewählt werden aus der Gruppe Phenoxyethanol, Methylparaben, Polyaminopropyl Biguanide, Benzethonium Chlorid und/oder Benzyl Alcohol.

Als Stabilisatoren sind im Sinne der vorliegenden Erfindung diejenigen Substanzen zu verstehen, die nicht in der Liste der zugelassenen Konservierungsstoffe (Kosmetikverordnung Anlage 6; Verordnung (EG) Nr. 1223/2009, Anhang V) aufgeführt sind, gleichwohl aber eine stabilisierende Wirkung haben und/oder im gemeinsamen Einsatz mit Konservierungsmitteln die Stabilität der Zubereitung 36 Monate fördern.

Als Stabilisatoren sind folgende Verbindungen vorteilhaft, die einzeln oder in Kombination eingesetzt werden können: Ethylhexylglycerin, 1,2-Hexanediol, Trisodium EDTA, Methylpropanediol, Caprylyl Glycol, Glyceryl Caprylate und/oder Pentylene Glycol.

Es ist darüber hinaus vorteilhaft, wenn wenigstens ein Konservierungsmittel und wenigstens ein Stabilisator in der erfindungsgemäßen Zubereitung enthalten sind.

In der erfindungsgemäßen Zubereitung liegt das wenigstens ein Konservierungsmittel und/oder der wenigstens eine Stabilisator mit einem Gehalt von 0,0001 bis 6 Gew. %, bevorzugt von 0,0005 bis 5 Gew. %, besonders bevorzugt von 0,001 bis 4 Gew. % vor, bezogen auf das Gesamtgewicht der Zubereitung.

Erfindungsgemäß vorteilhaft ist ebenfalls, wenn zusätzlich ein oder mehrere Feuchthaltemittel in die erfindungsgemäße Zubereitung eingearbeitet werden. Feuchthaltemittel können beispielsweise, der Haut Feuchtigkeit verleihen und/oder dazu beitragen, einen Feuchtigkeitsverlust der Haut zu verhindern. Bei den Feuchthaltemitteln handelt es sich um hygroskopische Substanzen, die Wasser binden können. Dieses Wasserbindevermögen beruht auf hydrophilen Gruppen, wie Hydroxylgruppen, aber auch Amingruppen und Carboxylgruppen. Feuchthaltemittel sind beispielsweise Propylenglycol, Butylenglycol, Glycerin, Sorbitol, Xylitol, Aloe Vera Gel. Erfindungsgemäß bevorzugt ist der Einsatz von Propylenglycol und/oder Glycerin.

Das Feuchthaltemittel oder die Feuchthaltemittel liegt/en mit einem Gesamtgehalt von 0,01 bis 10 Gew.-%, bevorzugt 0,1 bis 5,0 Gew.-% bezogen auf das Gesamtgewicht der Zubereitung, vor.

Erfindungsgemäß vorteilhaft ist ebenfalls, wenn zusätzlich ein oder mehrere Öle in die erfindungsgemäße Zubereitung eingearbeitet werden. Die Öle können ausgewählt werden aus der Gruppe der polaren Öle, beispielsweise aus der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamiaöl und dergleichen mehr.

Weitere vorteilhafte polare Ölkomponenten können im Sinne der vorliegenden Erfindung ferner gewählt werden aus der Gruppe der Monoester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Ethylhexylstearate, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölkomponente vorteilhaft gewählt werden aus der Gruppe der Dialkylether und Dialkylcarbonate, vorteilhaft sind z. B. Dicaprylylether (beispielsweise erhältlich unter der Handelsbezeichnung Cetiol OE) und/oder Dicaprylylcarbonat (beispielsweise erhältlich unter der Handelsbezeichnung Cetiol CC).

Auch beliebige Abmischungen der genannten Ölkomponenten sind vorteilhaft im Sinne der vorliegenden Erfindung einzusetzen.

Besonders bevorzugt sind Öle wie Sonnenblumenöl, Rizinusöl, Sojaöl, Jojobaöl, Traubenkernöl, Mandelöl und Macadamiaöl, die als pflegende Öle bezeichnet werden können.

Das Öl oder die Öle liegt/en vorteilhaft mit einem Gesamtgehalt von 0,01 bis 3,0 Gew.-%, bevorzugt 0,05 bis 2,0 Gew.-%, bezogen auf das Gesamtgewicht der Zubereitung, vor.

Die Einarbeitung wenigstens eines Öls, insbesondere eines natürlichen, pflanzlichen Öls in die erfindungsgemäße Zubereitung trägt dazu bei, eine Pflegeleistung zu erzielen oder zu verbessern. Gleichzeitig wird die Reinigungsleistung der erfindungsgemäßen Zubereitung verstärkt.

Die erfindungsgemäße Zubereitung kann vorteilhaft Parfüm enthalten. In der Regel ist das eingesetzte Parfüm eine Mischung aus Parfümrohstoffen. Das Parfüm ist hauptsächlich enthalten, um der erfindungsgemäßen Zubereitung einen angenehmen Geruch zu verleihen.

In der erfindungsgemäßen Zubereitung liegt das Parfüm mit einem Gehalt von 0,05 bis 0,5 Gew.-%, insbesondere 0,1 bis 0,4 Gew.-%, bezogen auf das Gesamtgewicht der Zubereitung vor.

Die erfindungsgemäße Zubereitung kann kosmetische Hilfsstoffe enthalten, wie sie üblicherweise in solchen Zubereitungen verwendet werden, z.B. Komplexbildner oder andere übliche Bestandteile einer kosmetischen Formulierung.

Zur pH-Werteinstellung werden beispielsweise Natriumhydroxid oder Citronensäure verwendet. Der pH-Wert der erfindungsgemäßen Zubereitungen liegt im Bereich 5.0 bis 8.0, bevorzugt im Bereich 6.5 bis 7.5.

Um zu zeigen, dass die vorstehende Aufgabe gelöst wurde, wurde eine Sedimentations-Studie durchgeführt. Beispielhaft wurde das Pigment Sicovit Brown 70 E172 (Venator Pigments), eine Mischung aus den Eisenoxiden CI 77492, CI77491 und CI 77499, in verschiedene Tensidlösungen, die keine verdickenden Zusätze enthielten, eingebracht. Üblicherweise kann das genannte Pigment wird in Make-up-Zubereitungen eingearbeitet werden, wie beispielsweise Foundations und Mascaras. Hierbei wurde durch starkes Schütteln eine homogene, trübe Suspension der Eisenoxide hergestellt und die Trübung (bzw. Sedimentation) nach unterschiedlichen Zeitpunkten vermessen.

Folgende Tensidlösungen wurden hergestellt:
1.) 0,1% Lauryl Ethersulfat mit 8 mg Pigment (Abkürzung LES)
2.) 0,1% Decyl Glucoside mit 8 mg Pigment (Abkürzung DG)
3.) 0,1% Rhamnolipid mit 8 mg Pigment (Abkürzung RHL)
4.) Wasser mit 8 mg Pigment (Vergleichslösung, Abkürzung H2O).

Die Proben wurden in einem Photometer (Analytik Jena, Specord 250) bei 600 nm vermessen. Es wurde eine 1 cm Küvette 3,4 cm hoch mit frisch aufgeschüttelter Pigmentlösung befüllt und sofort vermessen. Im Abstand von 30 Minuten (zuzüglich jeweils 30 Sekunden Messzeit) wurden 59 weitere Messungen je Probe durchgeführt. Der Mess-Lichtstrahl befand sich in 2,1 cm Höhe. Die Sedimentation der Pigmente kann über die Zunahme der Licht-Transmission nachverfolgt werden.

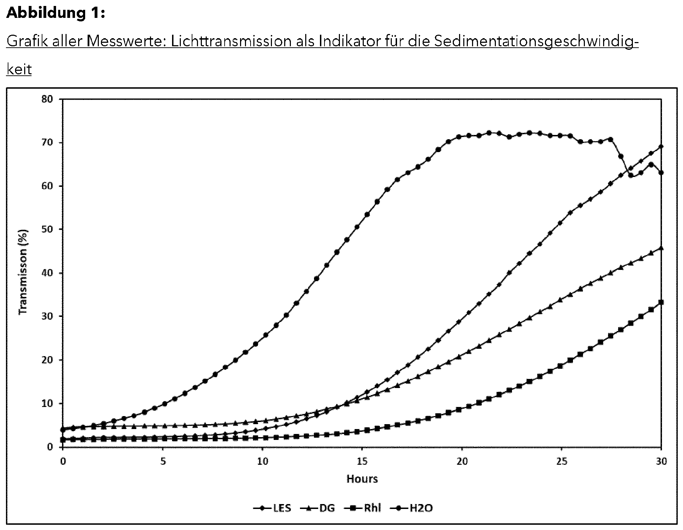

Die Ergebnisse wurden in der nachfolgenden Tabelle zusammengefasst und in einer Graphik dargestellt (siehe Abbildung 1).

**Tabelle: Lichttransmission als Indikator für die Sedimentationsgeschwindigkeit**

| | **Start** | **5 h** | **10 h** | **15 h** | **20 h** | **25 h** | **30 h** |
|---|---|---|---|---|---|---|---|
| | **T (%)** | **T (%)** | **T (%)** | **T (%)** | **T (%)** | **T (%)** | **T (%)** |
| **0,1% Lauryl Ethersulfat** | 1,9 | 2,4 | 4,3 | 12,6 | 28,7 | 51,5 | 69,1 |
| **0,1% Decyl Glucoside** | 4,3 | 4,9 | 6,1 | 11,4 | 20,7 | 33,8 | 45,8 |
| **0,1% Rhamnolipid** | 1,7 | 1,9 | 2,2 | 3,9 | 8,6 | 18,6 | 33,2 |
| **Wasser** | 4,0 | 10,0 | 25,7 | 53,5 | 71,3 | 71,7* | 63,1* |

| | | | | | | | |
|---|---|---|---|---|---|---|---|
| * Bei Wasser war erkennbar, dass sich die Pigmente z.T. an der Glasscheibe der Küvette festsetzten und diese damit während des Messvorganges verschmutzten, was zu einer geringeren und schwankenden Transmission führte. | | | | | | | |

Es wird deutlich, dass die Tensidlösung enthaltend Rhamnolipid die geringste Zunahme der Transmissionswerte während des Messzeitraums zeigt. Dies bedeutet, dass die Pigmente am besten in Schwebe gehalten werden. Dies bedeutet auch, dass die Pigmente am bestem solubilisiert werden, im Vergleich zu den Tensidlösungen enthaltend Laurylethersulfat oder Decyl Glucosid oder im Vergleich zu Wasser. Dies zeigt damit in Bezug auf Pigmente eine bessere Reinigungswirkung der untersuchten Rhamnolipidlösung im Vergleich zu üblicherweise in Reinigungszubereitungen eingesetzten Tensiden an.

Die Ausbildung von Mizellen kann experimentell nachgewiesen werden. Eine mögliche und auch geeignete Methode ist beispielsweise die Reverse-CMC. Hierbei kann eine unverdünnte Probe eingesetzt werden. Die Probe wird schrittweise verdünnt und nach jedem Verdünnungsschritt wird die Oberflächenspannung gemessen.

### Beispiele:

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitung bezogen.

| **Beispiel:** | **1** | **2** | **3** | **4** | **5** | **6** | **7** |
|---|---|---|---|---|---|---|---|
| Rhamnolipid | 0,4 | | 0,5 | | 0,2 | 0,3 | 0,4 |
| Mannosylerythrolipid | | 0,3 | | 0,3 | | | |
| Disodium cocoyl glutamate | | 0,1 | 0,1 | | | | |
| Sodium cocoamphoacetate | | | | | 0,2 | | |
| Cocoamidopropylbetain | | | | | | 0,2 | |
| Cocoglucoside | | | | | | | 0,1 |
| Phenoxyethanol | 0,8 | | | 0,8 | 0,8 | 0,4 | |
| 1,2 Hexandiol | | 0,5 | | | | | 0,5 |
| Octandiol | | | 0,3 | | | | |
| Glycerylcaprylate | | | | | | 0,2 | |
| Benzthenonium Chlorid | | 0,04 | 0,04 | | | | |
| Ethylhexylglycerin | | | | | 0,3 | | |
| Benzyl alcohol | | | | 0,2 | | | 0,2 |
| Glycerin | 2 | 1 | 0,5 | 1 | 2 | | 1 |
| Traubenkernöl | 0,001 | | | | | | |
| Fragrance | 0,12 | 0,2 | | 0,1 | 0,2 | | |
| Wasser | | | | | | | |

## Patentansprüche

1. Reinigungszubereitung, wobei die Zubereitung ist eine kosmetische Zubereitung zur Reinigung der menschlichen Haut, insbesondere der Gesichtshaut ist, enthaltend
- ≤ 0,5 Gew.-%, bevorzugt ≤ 0,3 Gew.-% wenigstens eines Glycolipids bezogen auf das Gesamtgewicht der Zubereitung und bezogen auf den Aktivgehalt und
- zusätzlich wenigstens ein weiteres Tensid, ausgewählt aus Aminosäure-basierten Tensiden, amphoteren Tensiden und/oder nichtionischen Tensiden,
wobei dass das wenigstens eine Glycolipid und wenigstens ein weiteres Tensid in einer Gesamttensidmenge vorliegen, die ≤ 0,8 Gew. % ist, bezogen auf die Gesamtmenge der Zubereitung.

2. Reinigungszubereitung nach Anspruch 1, **dadurch gekennzeichnet, dass** das wenigstens eine Glycolipid ausgewählt wird aus der Gruppe Rhamnolipide, Sophorolipide, Mannosylerythrollipide und/oder Trehaloselipide, insbesondere Rhamnolipide.

3. Reinigungszubereitung nach wenigstens einem der vorstehenden Ansprüche, **dadurch gekennzeichnet, dass** das wenigstens eine Glycolipid, bevorzugt das wenigstens eine Rhamnolipid, mit einem Gehalt von 0,01 bis 0,5 Gew.-%, bevorzugt von 0,05 bis 0,3 Gew.-% vorliegt, bezogen auf die Gesamtmenge des Zubereitung und bezogen auf den Aktivgehalt.

4. Reinigungszubereitung nach wenigstens einem der vorstehenden Ansprüche, **dadurch gekennzeichnet, dass** wenigstens ein Konservierungsmittel enthalten ist, bevorzugt ausgewählt aus der Gruppe Phenoxyethanol, Methylparaben, Polyaminopropyl Biguanide, Benzethonium Chloride und/oder Benzylalkohol.

5. Reinigungszubereitung nach wenigstens einem der vorstehenden Ansprüche, **dadurch gekennzeichnet, dass** wenigstens ein Stabilisator ausgewählt aus der Gruppe Ethylhexylglycerin, 1,2-Hexanediol, Methylpropanediol, Caprylyl Glycol, Glyceryl Caprylate und/oder Pentylene Glycol enthalten ist.

6. Reinigungszubereitung nach wenigstens einem der vorstehenden Ansprüche, **dadurch gekennzeichnet, dass** wenigstens ein Konservierungsmittel und wenigstens ein Stabilisator enthalten ist.

7. Reinigungszubereitung nach wenigstens einem der vorstehenden Ansprüche, **dadurch gekennzeichnet, dass** das wenigstens eine Konservierungsmittel und/oder der wenigstens eine Stabilisator mit einem Gehalt von 0,0001 bis 6,0 Gew.-%, bevorzugt von 0,0005 bis 6,0 Gew. %, besonders bevorzugt von 0,001 bis 4,0 Gew.-% vorliegen, bezogen auf das Gesamtgewicht der Zubereitung.

8. Reinigungszubereitung nach wenigstens einem der vorstehenden Ansprüche, **dadurch gekennzeichnet, dass** das wenigstens eine Aminosäure-basierte Tensid ausgewählt ist aus Acylglutamaten und/oder Acylglycinaten.

9. Reinigungszubereitung nach wenigstens einem der vorstehenden Ansprüche, **dadurch gekennzeichnet, dass** das wenigstens eine amphotere Tensid ausgewählt ist aus
- Betainen, insbesondere Alkylamidopropylbetainen und/oder Alkylbetainen;
- Sultainen, insbesondere Hydroxysultaine und/oder Amidopropylhydroxysultaine und/oder
- Acylamphoacetaten.

10. Reinigungszubereitung nach wenigstens einem der vorstehenden Ansprüche, **dadurch gekennzeichnet, dass** das wenigstens eine nichtionische Tensid ausgewählt wird aus den Alkylpolyglycosiden und/oder Polyglycerinestern.

11. Reinigungszubereitung nach wenigstens einem der vorstehenden Ansprüche, **dadurch gekennzeichnet, dass** zusätzlich wenigstens ein Feuchthaltemittel, insbesondere ausgewählt aus Propylenglycol, Butylenglycol, Glycerin, Sorbitol, Xylitol, Aloe Vera Gel, weiter insbesondere mit einem Gesamtgehalt von 0,01 bis 10 Gew.-%, bevorzugt 0,1 bis 5,0 Gew. % bezogen auf das Gesamtgewicht der Zubereitung, enthalten ist.

12. Reinigungszubereitung nach wenigstens einem der vorstehenden Ansprüche, **dadurch gekennzeichnet, dass** zusätzlich wenigstens ein Öl, insbesondere ausgewählt aus Gruppe der synthetischen, halbsynthetischen und natürlichen Öle, weiter insbesondere ausgewählt aus der Gruppe Sonnenblumenöl, Rizinusöl, Sojaöl, Jojobaöl, Traubenkernöl, Mandelöl und/oder Macadamiaöl und ganz insbesondere mit einem Gesamtgehalt von 0,001 bis 3,0 Gew.-%, bevorzugt 0,01 bis 1,0 Gew.-%, bezogen auf das Gesamtgewicht der Zubereitung, enthalten ist.

## Claims

1. Cleansing preparation, wherein the preparation is a cosmetic preparation for cleansing the human skin, more particularly the skin of the face, containing
- ≤ 0.5 wt%, preferably ≤ 0.3 wt%, of at least one glycolipid, based on the total weight of the preparation and based on the active content, and
- additionally at least one further surfactant, selected from amino acid-based surfactants, amphoteric surfactants and/or nonionic surfactants,
wherein the at least one glycolipid and at least one further surfactant are present in a total surfactant amount of ≤ 0.8 wt%, based on the total amount of the preparation.

2. Cleansing preparation according to Claim 1, **characterized in that** the at least one glycolipid is selected from the group of rhamnolipids, sophorolipids, mannosylerythritol lipids and/or trehalose lipids, in particular rhamnolipids.

3. Cleansing preparation according to at least one of the preceding claims, **characterized in that** the at least one glycolipid, preferably the at least one rhamnolipid, is present with a content of 0.01 to 0.5 wt%, preferably of 0.05 to 0.3 wt%, based on the total amount of the preparation and based on the active content.

4. Cleansing preparation according to at least one of the preceding claims, **characterized in that** there is at least one preservative, preferably selected from the group of phenoxyethanol, methylparaben, polyaminopropyl biguanide, benzethonium chloride and/or benzyl alcohol.

5. Cleansing preparation according to at least one of the preceding claims, **characterized in that** there is at least one stabilizer selected from the group of ethylhexylglycerol, 1,2-hexanediol, methylpropanediol, caprylyl glycol, glyceryl caprylate and/or pentylene glycol.

6. Cleansing preparation according to at least one of the preceding claims, **characterized in that** there is at least one preservative and at least one stabilizer.

7. Cleansing preparation according to at least one of the preceding claims, **characterized in that** the at least one preservative and/or the at least one stabilizer are/is present with a content of 0.0001 to 6.0 wt%, preferably of 0.0005 to 6.0 wt%, more preferably of 0.001 to 4.0 wt%, based on the total weight of the preparation.

8. Cleansing preparation according to at least one of the preceding claims, **characterized in that** the at least one amino acid-based surfactant is selected from acylglutamates and/or acylglycinates.

9. Cleansing preparation according to at least one of the preceding claims, **characterized in that** the at least one amphoteric surfactant is selected from
- betaines, more particularly alkylamidopropylbetaines and/or alkylbetaines;
- sultaines, more particularly hydroxysultaines and/or amidopropylhydroxysultaines; and/or
- acylamphoacetates.

10. Cleansing preparation according to at least one of the preceding claims, **characterized in that** the at least one nonionic surfactant is selected from the alkylpolyglycosides and/or polyglycerol esters.

11. Cleansing preparation according to at least one of the preceding claims, **characterized in that** additionally there is at least one humectant, more particularly selected from propylene glycol, butylene glycol, glycerol, sorbitol, xylitol, aloe vera gel, more particularly still with a total content of 0.01 to 10 wt%, preferably 0.1 to 5.0 wt%, based on the total weight of the preparation.

12. Cleansing preparation according to at least one of the preceding claims, **characterized in that** additionally there is at least one oil, more particularly selected from the group of the synthetic, semi-synthetic and natural oils, more particularly still selected from the group of sunflower oil, castor oil, soya oil, jojoba oil, grape kernel oil, almond oil and/or macadamia oil and especially with a total content of 0.001 to 3.0 wt%, preferably 0.01 to 1.0 wt%, based on the total weight of the preparation.

## Revendications

1. Préparation nettoyante, la préparation étant une préparation cosmétique pour le nettoyage de la peau humaine, en particulier de la peau du visage, contenant
- ≤ 0,5% en poids, de préférence ≤ 0,3% en poids, d'au moins un glycolipide par rapport au poids total de la préparation et par rapport à la teneur en principe actif et
- en plus au moins un autre tensioactif, choisi parmi les tensioactifs à base d'aminoacides, les tensioactifs amphotères et/ou les tensioactifs non ioniques,
ledit au moins un glycolipide et au moins un autre tensioactif se trouvant en une quantité totale de tensioactif de 0,8% en poids, par rapport à la quantité totale de la préparation.

2. Préparation nettoyante selon la revendication 1, **caractérisée**
**en ce que** ledit au moins un glycolipide est choisi dans le groupe des rhamnolipides, des sophorolipides, des lipides de mannosyl-érythritol et/ou des lipides de tréhalose, en particulier des rhamnolipides.

3. Préparation nettoyante selon au moins l'une des revendications précédentes, **caractérisée en ce que** ledit au moins un glycolipide, de préférence ledit au moins un rhamnolipide, est présent en une teneur de 0,01 à 0,5% en poids, de préférence de 0,05 à 0,3% en poids, par rapport à la quantité totale de la préparation et par rapport à la teneur en principe actif.

4. Préparation nettoyante selon au moins l'une des revendications précédentes, **caractérisée en ce qu'**au moins un conservateur est contenu, de préférence choisi dans le groupe phénoxyéthanol, méthylparabène, polyaminopropyl-biguanide, chlorure de benzéthonium et/ou alcool benzylique.

5. Préparation nettoyante selon au moins l'une des revendications précédentes, **caractérisée en ce qu'**au moins un stabilisant, choisi dans le groupe éthylhexylglycérol, 1,2-hexanediol, méthylpropanediol, caprylylglycol, caprylate de glycéryle et/ou pentylèneglycol, est contenu.

6. Préparation nettoyante selon au moins l'une des revendications précédentes, **caractérisée en ce qu'**au moins un conservateur et au moins un stabilisant sont contenus.

7. Préparation nettoyante selon au moins l'une des revendications précédentes, **caractérisée en ce que** ledit au moins un conservateur et/ou ledit au moins un stabilisant est/sont présent(s) en une teneur de 0,0001 à 6% en poids, de préférence de 0,0005 à 6,0% en poids, de manière particulièrement préférée de 0,001 à 4,0% en poids, par rapport au poids total de la préparation.

8. Préparation nettoyante selon au moins l'une des revendications précédentes, **caractérisée en ce que** ledit au moins un tensioactif à base d'aminoacides est choisi parmi les acylglutamates et/ou les acylglycinates.

9. Préparation nettoyante selon au moins l'une des revendications précédentes, **caractérisée en ce que** ledit au moins un tensioactif amphotère est choisi parmi
- les bétaïnes, en particulier les alkylamidopropylbétaïnes et/ou les alkylbétaïnes ;
- les sultaïnes, en particulier les hydroxysultaïnes et/ou les amidopropylhydroxysultaïnes et/ou
- les acylamphoacétates.

10. Préparation nettoyante selon au moins l'une des revendications précédentes, **caractérisée en ce que** ledit au moins un tensioactif non ionique est choisi parmi les alkylpolyglycosides et/ou les esters de polyglycérol.

11. Préparation nettoyante selon au moins l'une des revendications précédentes, **caractérisée en ce qu'**en plus au moins un agent hydratant, en particulier choisi parmi le propylèneglycol, le butylèneglycol, le glycérol, le sorbitol, le xylitol, le gel d'aloe vera, en outre en particulier en un teneur totale de 0,01 à 10% en poids, de préférence de 0,1 à 5,0% en poids, par rapport au poids total de la préparation, est contenu.

12. Préparation nettoyante selon au moins l'une des revendications précédentes, **caractérisée en ce qu'**en plus au moins une huile, en particulier choisie dans le groupe des huiles synthétiques, semi-synthétiques et naturelles, en outre en particulier choisie dans le groupe huile de tournesol, huile de ricin, huile de soja, huile de jojoba, huile de pépins de raisin, huile d'amande et/ou huile de noix de macadamia et tout particulièrement en une teneur totale de 0,001 à 3,0% en poids, de préférence de 0,01 à 1,0% en poids, par rapport au poids total de la préparation, est contenue.
